Europäisches Patentamt

European Patent Office

Office européen des brevets

(11) Publication number: **0 338 081**
**A1**

## EUROPEAN PATENT APPLICATION
### published in accordance with Art. 158(3) EPC

(21) Application number: 88908344.0

(22) Date of filing: 21.09.88

(86) International application number:
PCT/JP88/00953

(87) International publication number:
WO 89/02732 (06.04.89 89/08)

(51) Int. Cl.⁴: **A61K 9/10**

(30) Priority: 25.09.87 JP 241958/87

(43) Date of publication of application:
**25.10.89 Bulletin 89/43**

(84) Designated Contracting States:
**AT BE CH DE FR GB IT LI LU NL SE**

(71) Applicant: **FUJISAWA PHARMACEUTICAL CO., LTD.**
**3, Doshomachi 4-chome Higashi-ku**
**Osaka-shi Osaka 541(JP)**

(72) Inventor: UEDA, Yoshio
**3-5-204, Mikagenakamachi 1-chome**
**Higashinada-ku**
**Kobe-shi Hyogo 658(JP)**

Inventor: **HATA, Takehisa**
**1-2, Kayohgaoka 2-chome**
**Nagaokakyo-shi Kyoto 617(JP)**
Inventor: **KIMURA, Sumihisa**
**13-1-408, Minamihibarigaoka 2-chome**
**Takarazuka-shi Hyogo 665(JP)**
Inventor: **KOHNO, Yutaka**
**12-1-508, Aoba 3-chome -himamoto-cho**
**Mishima-gun Osaka 618(JP)**

(74) Representative: **Türk, Dietmar, Dr. rer. nat. et al**
**Türk, Gille + Hrabal Patentanwälte Bruckner**
**Strasse 20**
**D-4000 Düsseldorf 13(DE)**

(54) **OILY RECTAL INSTILLATION.**

(57) Oily preparation for intrarectal infusion Contains water-soluble drug, dispersing agent and surfactant in which HLB value is 10 or more.

It can prevent the water-soluble drug from precipitating in the container for infusion, even in case that the concentration of the water-soluble drug and/or the dispersing agent is high, and can easily be filled in the container for infusion and can easily be administered to the patient, and further the stability and the bioavailability of the water-soluble drug and its matabolic inhibitor in the preparation are both good.

## OILY PREPARATION FOR INTRARECTAL INFUSION

TECHNICAL FIELD

This invention relates to an oily preparation for intrarectal infusion containing water-soluble drug, dispersing agent and surfactant in which HLB value is 10 or more and this invention is utilized in the therapeutical field.

BACKGROUND ART

Although suppositories are widely used as the preparation for intrarectal administration, they have many problems.

Namely, the suppositories can not easily control the dose, are ruined in shape under relatively high temperature, contaminate hand(s) when administered to the patients, are felt like foreign substances in the rectum until they are dissolved since they are solid form, can not easily be administered to the patient whose sphincter ani relaxes, etc.

The rectal capsules, wherein the inner solution in which the drug is dissolved or suspended in oily substance is covered with the gelatin film containing the plasticizer such as glycerin, have already been developed as the preparation which has improved said one disadvantage of suppositories that are ruined in shape under relatively high temperature.

However, the rectal capsules have some disadvantages mentioned above.

Namely, the rectal capsules contaminate hand(s) when administered to the patients, can not easily control the dose, are felt like foreign substances in the rectum until they are dissolved, can not easily be administered to the patient whose sphincter ani relaxes, etc.

The inventors of the present invention have already developed the oily suspension for intrarectal infusion, wherein water-soluble drug and gelling agent are suspended in oily substance by using dispersing agent, as the preparation for intrarectal administration which has solved the above these problems (Japanese Patent Unexamined Publication No. 45521/1987).

However, as the above oily suspension for intrarectal infusion was filled in the container which was suitable for the intrarectal administration, in case the drug was precipitated in the container, the drug was remained in the container and the uniform dose could not be obtained when it was administered.

Though the dispersing agent was added in the preparation in order to prevent the drug from precipitating, the drug was precipitated in the container in case the drug concentration in the preparation was high or the drug was precipitated due to the property of the drug even if the drug concentration in the preparation was low.

It was necessary to add the dispersing agent in high concentration in the preparation in order to prevent the drug from precipitating in said cases.

However, there were problems that such preparation could not be easily filled in the container for infusion and could not be easily administered to the patient (it is said that suitable viscosity for the filling to the container and the administration to the patient is about 1,000-20,000 cps), because the fluidity of the preparation was bad due to the high viscosity of the preparation.

DISCLOSURE OF THE INVENTION

The inventors of this invention have carried out extensive studies in order to overcome the above-mentioned problems and have found that the oily preparation for intrarectal infusion containing water-soluble drug, dispersing agent and surfactant in which HLB value is 10 or more could prevent the water-soluble drug from precipitating in the container for infusion, even in case that the concentration of the water-soluble drug and/or the dispersing agent was high, and could easily be filled in the container for infusion and could easily be administered to the patient because it could retain a suitable viscosity and the fluidity was good, and further the stability and the bioavailability of the water-soluble drug and its matabolic inhibitor in the preparation were both good.

## DESCRIPTION OF THE PREFERRED EMBODIMENTS

The water-soluble drugs to be used in the present invention are usually the ones which are poorly absorbed from the gastro-intestinal tract.

Suitable water-soluble drug may be, for example, cephalospolin antibiotics (e. g. ceftizoxime, cefazolin, cephaloglycin, cephalothin, cephaloridine, their sodium salts, etc.), penicillin antibiotics (e. g. benzylpenicillin, carbenicillin, sulbenicillin, piperacillin, their sodium salts, etc.), amino glucoside antibiotics (e. g. gentamycin, streptomycin, kanamycin, paromomycin, fradiomycin, sisomicin, etc.), anti tumor substances (e. g. fluorouracil, tegafur, mitomycin, adriamycin, bleomycin, etc.), peptide hormone (e.g. insulin, calcitonin, secretin, gastrin, somatomedin, α-human atrial natriuretic polypeptide (hereinafter referred to as "α-hANP"), etc.) or the like, but is not restricted to the above-mentioned drugs.

The dispersing agents to be used in the present invention may be the ones which have viscosity when dissolved or suspended in the oily substance.

Suitable dispersing agent may be, for example, surfactant such as glycerin mono fatty acid ester [e.g. MGS-B (brand name; prepared by Nikko Chemicals Co., Ltd.], dextrin fatty acid ester [e.g. Rheopearl KE (trademark; prepared by Kaihatsu Kagaku Co., Ltd.)], sucrose fatty acid ester, sorbitan fatty acid ester, polyoxyethylene sorbitol fatty acid ester, polyoxyethylene fatty acid ester, polyoxyethylene higher alcohol ether, polyoxyethylene alkylaryl ether, propyleneglycol mono fatty acid ester, polyoxyethylene castor oil drivatives, polyoxypropylenepolyoxyethylene cetylalcohol ester or the like, aluminum stearate, light anhydrous silicic acid, etc. Among these dispersing agents, the surfactants which have low HLB value (HLB value: about 3-7) are preferable.

These dispersing agents can prevent the water-soluble drug contained in the oily preparation for intrarectal infusion from precipitating in the container for infusion.

Suitable surfactant in which HLB value is 10 or more may be, for example, the one in which HLB value is 10 or more, such as polyoxyethylene sorbitol fatty acid ester [e.g. TO-10 (brand name; prepared by Nikko Chemicals Co., Ltd.)], polyoxyethylene polyoxypropyleneglycol [e.g. PLURONIC F-68 (brand name; prepared by BASF Co., Ltd.)], polyethyleneglycol fatty acid ester [e.g. MYS-40 (brand name; prepared by Nikko Chemicals Co., Ltd.)], polyoxyethylene alkyl ether [e.g. BL-9Ex (brand name; prepared by Nikko Chemicals Co., Ltd.), BO-20 (brand name; prepared by Nikko Chemicals Co., Ltd.)], polyoxyethylene alkylaryl ether [e.g. NP-10 (brand name; prepared by Nikko Chemicals Co., Ltd.)], polyoxyethylene castor oil derivatives [e.g. HCO-60 (brand name; prepared by Nikko Chemicals Co., Ltd.)], sucrose fatty acid ester [e.g. DK ester F-160 (brand name; prepared by Dai-ichi Kogyo Seiyaku Co., Ltd.] etc.

Among these surfactants, the surfactants in which HLB value is 12 or more are preferable and the surfactants in which HLB value is 14 or more are more preferable.

As these surfactants in which HLB value is 10 or more can improve the fluidity of the oily preparation for intrarectal infusion with preventing the precipitation of the water-soluble drug, they have the effects that the preparation can easily be filled in the container for infusion and can easily be administered to the patient.

Further, they have the effect that the water-soluble drug can easily be dissolved from the oily substance.

In case that the water-soluble drug is poorly absorbed from the gastrointestinal tract, it is desirable to add absorption-promoter.

Suitable absorption-promoter may be the conventional one such as bile acid (e.g. cholic acid, glycocholic acid, taurocholic acid, deoxycholic acid, glycodeoxycholic acid, taurodeoxycholic acid, chenodeoxycholic acid, glycochenodeoxycholic acid, taurochenodeoxycholic acid, ursodeoxycholic acid, glycoursodeoxycholic acid, tauroursodeoxycholic acid, lithocholic acid, glycolithocholic acid, taurolithocholic acid, dehydrocholic acid, taurodehydrocholic acid, etc.) and their alkali metal salts, surfactant [e.g. Span 60 (trademark; prepared by Atras Powder Co., Ltd.), etc.], fatty acids (e.g. sodium caprate, etc.), chelate (e.g. EDTA, etc.), alkaline earth metal salts of aldonic acid (e.g. calcium gluconate, etc.), salicylic acid, flufenamic acid or the like.

Among these absorption-promoters, cholic acid glycocholic acid, taurocholic acid, chenodeoxycholic acid or glycochenodeoxycholic acid is more preferable due to weak rectal irritation and strong effect on absorption-enhancement.

The ratio of water-soluble drug, dispersing agent and surfactant in which HLB value is 10 or more in the oily preparation for intrarectal infusion of the present invention is usually 0.01-50 weight %, 0.1-10 wieght % and 0.01-10 weight % respectively, but is not restricted to the above ratio and is optionally determined. Preferable ratio of these components is shown in the below-mentioned Examples.

The oily preparation for intrarectal infusion of the present invention may contain metabolic inhibitor for

3

above-mentioned water-soluble drugs (such as, ß-lactamase inhibitor for cephalospolin antibiotics, uracil for tegafur, etc.) and conventional additives [e.g. gelling agents, antioxidants, antiseptics, dissolving aids for the drugs (such as, nicotinamide, urea, etc), etc.].

The oily preparation for intrarectal infusion of the present invention can be prepared by mixing and dispersing the above-mentioned components with the oily substance.

Oily substance to be used in the present invention may be the one such as vegetable fatty oil (e.g. olive oil, soybean oil, sesami oil, safflower oil, rape seed oil, etc.), animal fatty oil (e.g. mink oil, egg oil, squalene, etc.), mono-, di- or triglyceride(s) of middle chain -saturated fatty acid [e.g. Coconad MT (trademark; prepared by Kao Food Co., Ltd.), Miglyol 812 (trademark; prepared by Dynamit Novel Co., Ltd.), MGK (brand name; prepared by Nikko Chemicals Co., Ltd.), etc.], higher fatty acid (e.g. olelic acid, etc.), liquid lanolin, squalane or the like.

The oily preparation for intrarectal infusion thus obtained is filled into the container which is suitable for intrarectal administration (e.g. the container which is disclosed in Japanese Utility Model Unexamined Publication No. 50344/1988) and used. To illustrate the effect of the present invention, test examples are shown in the following.

Stability test

(i) Test method

The preparation obtained in Example 1 was filled in a glass vial and the sample was in a pyrostat at 40° C and the stability test was conducted.

Appearance, content of the drug (measured by high performance liquid chromatography) and viscosity (measured by EHD type rotated viscometer, 25° C, 0.5 rpm) of the preparation were measured.

(ii) Test results

| | | Appearance | Content of the drug (%) | Viscosity (cps) |
|---|---|---|---|---|
| | Initial | White suspension having lustered surface | 100.0 | 14470 |
| 40°C | 1 month | White suspension with clear separated solution slightly at the surface | 98.0 | 14590 |
| | 2 months | White suspension with clear separated solution slightly at the surface | 98.4 | 14690 |

5

From the above test results, it is found that the drug in the oily preparation for intrarectal infusion of the present invention is hardly precipitated when placed at 40° C for 2 months and the content of the drug and viscosity in the preparation are both stable.

Bioavailability test 1

(i) Test method

The oily preparation for intrarectal infusion obtained in Example 1 and tegafur suppository (brand name : Ftorafur Zupo,prepared by Taiho Pharmaceutical Co., Ltd.) were administered rectally to five male Wister rats fasted overnight. The dose was 34 mg as tegafur respectively.

0.5, 1, 2, 4, 6 and 8 hours after rectal administration, blood samples were collected and plasma concentrations of tegafur were measured by high-performance liquid chromatography.

(ii) Test results

|  | mean plasma concentration (μg/ml) | | | | | |
|---|---|---|---|---|---|---|
|  | 0.5 hour | 1 hour | 2 hours | 4 hours | 6 hurs | 8 hours |
| Oily preparation for intrarectal infusion (Example 1) | 127 | 181 | 229 | 205 | 158 | 119 |
| Tegafur suppository | 133 | 196 | 214 | 179 | 139 | 116 |

From the above test results, it is found that the oily preparation for intrarectal infusion of the present invention has the same or superior bioavailability as compared with tegafur suppository.

Bioavailability test 2

(i) Test method

The oily preparation for intrarectal infusion obtained in Example 6 was filled in the container for intrarectal infusion disclosed in Japanese Utility Model Unexamined Publication No. 50344/1988 and was administered rectally to nine male Beagle fasted overnight. The dose was 5.32 g (0.448 g as uracil).

0, 7.5, 15, 30 and 60 minutes after rectal administration, blood samples were collected and plasma concentrations of uracil were measured by high-performance liquid chromatography.

AUC

from 0 to 60 minutes after administration was calculated by trapezoidal method.

(ii) Test results

| | plasma concentration of uracil (μg/ml) | | | | | AUC (0-60 minutes) (μg.min/ml) |
|---|---|---|---|---|---|---|
| | 0 minute | 7.5 minutes | 15 minutes | 30 minutes | 60 minutes | |
| Oily preparation for intrarectal infusion (Example 6) | 0.26 ±0.08 | 2.99 ±0.66 | 3.30 ±1.06 | 2.47 ±1.19 | 0.84 ±0.39 | 128.83 ±48.36 |
| (mean ± S.E.) | | | | | | |

From the above test results, it is found that uracil which is added as a metabolic inhibitor for tegafur in the oily preparation for intrarectal infusion of the present invention is very absorbable.

The present invention is illustrated according to the examples as shown below.

Example 1

| tegafur | 750 mg |
|---|---|
| MGS-B | 40 mg |
| TO-10 | 6 mg |
| Miglyol 812 | suitable amount |
| total | 2000 mg |

MGS-B and TO-10 were added to Miglyol 812 at 70° C and then tegafur was kneaded and dispersed in the mixture to give an oily preparation for intrarectal infusion.

Example 2

| ceftizoxime sodium | 250 mg potency |
|---|---|
| MGS-B | 75 mg |
| TO-10 | 7.5 mg |
| cholic acid | 112.5 mg |
| Miglyol 812 | suitable amount |
| total | 2500 mg |

Oily preparation for intrarectal infution was obtained according to a similar procedure to that of Example 1.

Example 3

| fluorouracil | 37.5 mg |
|---|---|
| MGS-B | 30 mg |
| TO-10 | 3.6 mg |
| cholic acid | 48 mg |
| Miglyol 812 | suitable amount |
| total | 1200 mg |

Oily preparation for intrarectal infusion was obtained according to a similar procedure to that of Example 1.

Example 4

| insulin | 50 international units |
|---|---|
| MGS-B | 75 mg |
| TO-10 | 7.5 mg |
| cholic acid | 112.5 mg |
| Miglyol 812 | suitable amount |
| total | 2500 mg |

Oily preparation for intrarectal infusion was obtained according to a similar procedure to that of Example 1.

Example 5

| $\alpha$-hANP | 0.25 mg |
|---|---|
| MGS-B | 60 mg |
| TO-10 | 6 mg |
| cholic acid | 80 mg |
| Coconad MT | suitable amount |
| total | 2000 mg |

Oily preparation for intrarectal infusion was obtained according to a similar procedure to that of Example 1.

Example 6

| tegafur | 3.76 g |
|---|---|
| uracil | 8.43 g |
| MGS-B | 2.0 g |
| BO-20 | 2.0 g |
| nicotinamide | 6.0 g |
| cholic acid | 4.0 g |
| Miglyol 812 | suitable amount |
| total | 100 g |

8

MGS-B and BO-20 were added to Miglyol 812 at 70°C and then tegafur, uracil, nicotinamide and cholic acid were kneaded and dispersed in the mixture to give an oily preparation for intraractal infusion.

## Claims

1. Oily preparation for intrarectal infusion containing water-soluble drug, dispersing agent and surfactant in which HLB value is 10 or more.

2. Oily preparation for intrarectal infusion according to claim 1, wherein water-soluble drug is tegafur.

# INTERNATIONAL SEARCH REPORT

International Application No PCT/JP88/00953

| I. CLASSIFICATION OF SUBJECT MATTER (if several classification symbols apply, indicate all) 6 |
|---|

According to International Patent Classification (IPC) or to both National Classification and IPC

Int.Cl$^4$    A61K9/10

## II. FIELDS SEARCHED

Minimum Documentation Searched 7

| Classification System | Classification Symbols |
|---|---|
| IPC | A61K9/08, A61K9/10, A61K9/02 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| X | JP, A, 49-50119 (Takeda Chemical Industries, Ltd.) 15 May 1974 (15. 05. 74) Page 1, left column, lines 5 to 9, page 2, left column, lower part, line 20 to page 2, right column, lower part, line 4 & DE, A1, 2347227 & FR, B1, 2199998 | 1-2 |
| X | JP, A, 52-87218 (Yamanouchi Pharmaceutical Co., Ltd.) 20 July 1977 (20. 07. 77) Page 1, left column, lines 5 to 8, page 3, right column, lower part, line 19 to page 4, left column, upper part, line 3 (Family: none) | 1-2 |
| X | JP, A, 53-6417 (Yamanouchi Pharmaceutical Co., Ltd.) 20 January 1978 (20. 01. 78) Page 1, left column, lines 4 to 7, | 1-2 |

* Special categories of cited documents: 10

"A" document defining the general state of the art which is not considered to be of particular relevance

"E" earlier document but published on or after the International filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| November 10, 1988 (10. 11. 88) | November 21, 1988 (21. 11. 88) |
| International Searching Authority | Signature of Authorized Officer |
| Japanese Patent Office | |

Form PCT/ISA/210 (second sheet) (January 1985)    See notes on accompanying sheet

International Application No. PCT/JP88/00953

## FURTHER INFORMATION CONTINUED FROM THE SECOND SHEET

| X | JP, A, 55-81812 (Kowa Corp.) 20 June 1980 (20. 06. 80) Page 1, left column, lines 5 to 8, page 2, left column, lower part, lines 15 to 20 (Family: none) | 1 - 2 |
| Y | Osaka-fu Byoin Yakuzaishi-kai Henshu [Zentei Iyakuhin Yoran] 10. November 1983 (10. 11. 83) Yakugyo Jiho-sha Kabushiki Kaisha P.1066 | 2 |

### V.☐ OBSERVATIONS WHERE CERTAIN CLAIMS WERE FOUND UNSEARCHABLE[10]

This international search report has not been established in respect of certain claims under Article 17(2) (a) for the following reasons:

1.☐ Claim numbers_____ because they relate to subject matter[12] not required to be searched by this Authority, namely:

2.☐ Claim numbers_____, because they relate to parts of the international application that do not comply with the prescribed requirements to such an extent that no meaningful international search can be carried out[13], specifically:

### VI.☐ OBSERVATIONS WHERE UNITY OF INVENTION IS LACKING[11]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (October 1981)

See notes on accompanying sheet

International Application No. PCT/JP88/00953

| FURTHER INFORMATION CONTINUED FROM THE SECOND SHEET | | |
|---|---|---|
| . | page 3, right column, upper part, lines 2 to 8 (Family: none) | |
| X | JP, A, 53-20417 (Yamanouchi Pharmaceutical Co., Ltd.) 24 February 1978 (24. 02. 78) Page 1, left column, lines 5 to 10, page 3, left column, upper part, line 16 to page 3, right column, upper part, line 1 (Family: none) | 1 - 2 |

**V.☐ OBSERVATIONS WHERE CERTAIN CLAIMS WERE FOUND UNSEARCHABLE [10]**

This international search report has not been established in respect of certain claims under Article 17(2) (a) for the following reasons:

1.☐ Claim numbers_____, because they relate to subject matter [12] not required to be searched by this Authority, namely:

2.☐ Claim numbers_____, because they relate to parts of the international application that do not comply with the prescribed require-ments to such an extent that no meaningful international search can be carried out [13], specifically:

**VI.☐ OBSERVATIONS WHERE UNITY OF INVENTION IS LACKING [11]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (October 1981)

See notes on accompanying sheet